Europäisches Patentamt

European Patent Office

Office européen des brevets

(11) Publication number: **0 177 352**
A1

# EUROPEAN PATENT APPLICATION

(21) Application number: 85307079.5

(22) Date of filing: 03.10.85

(51) Int. Cl.⁴: **G 01 N 33/545**
G 01 N 33/535, G 01 N 33/04

(30) Priority: 04.10.84 AU 7502/84

(43) Date of publication of application:
09.04.86 Bulletin 86/15

(84) Designated Contracting States:
AT BE CH DE FR GB IT LI LU NL SE

(71) Applicant: THE STATE OF VICTORIA
Treasury Place
Melbourne Victoria 3000(AU)

(72) Inventor: Spencer, Terence Leslie
30 Garden Street
Benalla Victoria 3672(AU)

(72) Inventor: Patterson, Ronald Mark
"Victoree" Midland Highway
Benalla Victoria 3672(AU)

(74) Representative: Heath, Peter William Murray
FRY HEATH & CO. Seloduct House 16-18 Station Road
Redhill Surrey RH1 1NF(GB)

(54) Improvements in or relating to enzyme-linked immunosorbent assay.

(57) An apparatus for enzyme-linked immunosorbent assay including a vessel including a first line of wells; and a second line of wells; the first line of wells including at least one well adapted to form a control and at least one well coated with a first specific antigen, antibody or like material; the second line of wells (b) including at least one well adapted to form a control and at least one well coated with a second specific antigen, antibody or like material, each specific antigen, antibody or like material being selected to recognize and bind to a sample.

## Fig.1.

KEY:-
▨ – BEEF/BUFFALO
▤ – SHEEP
▥ – PIG
▦ – HORSE/DONKEY
▧ – KANGAROO
▨ – BUFFALO
▭ – GOAT
▩ – DONKEY

EP 0 177 352 A1

IMPROVEMENTS IN OR RELATING TO ENZYME-LINKED IMMUNOSORBENT ASSAY.

The present invention relates to an improved assay method and apparatus, particularly a method for detecting and identifying contaminents in a sample.

Testing samples for contamination by extraneous species and materials is an ongoing problem for the food industries. For example, substitution of sheep, horse and kangaroo meats, and the like, in Australian beef exports is a problem for the meat industry. Similarly, the substitution of cows milk for goats milk is a problem for the dairy industry.

In the prior art the majority of species monitoring has been performed by the Ouchterlony Immuno Diffusion Method. Whilst this method is adequate for the task the Ouchterlony test has significant disadvantages. Firstly, the Immuno Diffusion-type test is capable of detecting approximately 10% contamination. Contamination at a lower level than this is detectable only with difficulty. Further, the Ouchterlony test requires overnight development samples and uses large amounts of specific antiserum. This leads to a relatively high cost per test and is compounded by the inability to automate the test procedures.

Alternative methods utilized in the prior method including rocket immunoelectrophoresis (IEP), heamagglutination inhibition (HAI), isoelectric focusing (IEF) and radioimmunoassay (RIA) are all prohibitively expensive and complex for routine species monitoring.

It is accordingly an object of the present invention to overcome or at least alleviate one or more of the difficulties related to the prior art.

Accordingly, in a first aspect of the present invention there is provided an enzyme-linked immunosorbent assay (ELISA) apparatus including

1)      a vessel including

        (a)   a first line of wells; and

        (b)   a second line of wells;

        The first line of wells (a) may include

        (i)   at least one well adapted to form a control

and

        (ii)   at least one well coated with a first specific antigen, antibody or like material;

        The second line of wells (b) may include

        (i)   at least one well adapted to form a control

and

        (ii)   at least one well coated with a second specific antigen, antibody or like material.

        The enzyme linked immunosorbent assay according to the present invention has the advantage that it can be performed rapidly, typically 2-3 hours compared to 16 hours for Ouchterlony.  Moreover, the ELISA test provides substantially increased sensitivity.  The increased sensitivity enables, in a preferred aspect, the bulking of up to five samples for preliminary screening purposes. Individual samples need only then be tested if the bulk test produces a positive result.

        The vessel (1) according to the present invention may be a microtitre plate.

        The microtitre plate according   to the present invention may be of the "U" bottom type.  The microtitre plate may be manufactured in polystyrene or similar material.

        In a preferred form, the present invention provides

(c)      at least one further line of wells including

(i)   at least one well adapted to form a control and

(ii)   at least one well coated with a further specific antigen, antibody or like material.

Each lines of wells may be arranged as horizontal rows or vertical columns.  Each line of wells may include as many wells as are desired.  In a preferred embodiment each line may include from approximately 8-12 wells although the minimum number is 2.

The microtitre plate according to the present invention may include as many lines of wells as are desired. In a preferred aspect the microtitre plate may include from 2-8 lines depending on the number of species to be tested.

In a preferred form the microtitre plate may be arranged for multiple-test use so that any multiple of the well lines may be present on a single plate.

Accordingly, in a preferred aspect the present invention provides an ELISA apparatus including

1)   a first vessel of the type described above and

2)   at least one further vessel adjacent thereto.

The vessels may take the form of microtitre plates.

The microtitre plates 1 and 2 may be formed integrally.  Each microtitre plate may be separated from its adjacent plate in any suitable manner.  Accordingly the ELISA apparatus according to the present invention may further include

3)   a first dividing member between adjacent vessel.

The first dividing member (3) may comprise a raised portion between adjacent plates. The first dividing member may comprise an indented portion between adjacent plates.

According to a further aspect, the ELISA apparatus according to the present invention may include

4)   a second dividing member between the at least one coated well and the control well.

The second dividing member may be similar to the first dividing member. However it will be understood that the second dividing member will run perpendicularly to the said first dividing means.

As described above the first line of wells includes

1)       at least one well coated with a first specific antigen, antibody, or like material.

The first specific antigen, antibody or like material may be selected from those which recognize and bind to a sample. The antigen, antibody or like material may be selected to recognize and bind with serum components in the sample, non-serum blood components contained in the sample, muscle constituents or the like.

The antigens, antibodies or like material may be produced by immunizing an animal with a serum from a species to be tested. The animal may be selected from rabbits, sheep, goats, or chickens. Alternatively the antibodies may be produced by synthetic means. Monoclona/antibody technology may be used. For example a specific anti-sheep reagent may be prepared by immunizing a goat with approximately 20 ml of whole sheep serum in a multiple emulsion of Frenuds adjuvant.

The specific anti-species reagents may be purified utilising affinity chromatography procedures. If cross-reactions occur, further purification may be achieved by absorption on a further serum affinity column of the cross-reacting species.

If anti-idiotypic antibodies are produced these may be removed by passing the appropriate affinity extracted antibody through a serum affinity column of the same species from which that antibody was prepared, e.g. goat-antisheep would have to be passed through a goat serum affinity column.

The sample to be tested may be selected from protein-containing materials. The sample may be a meat, a milk or products thereof.

In a preferred aspect the sample to be tested may be a sample of meat. The antigens, antibodies or like materials may be selected to recognize beef, sheep and substitutes therefor. In a preferred form the microtitre plate according to the present invention may include 8 lines of wells coated with 8 specific antigens, antibodies or like materials. The 8 specific antigens, antibodies or like materials may be selected from 8 species specific antisera. The antisera may include a beef/buffalo -specific antiserum, a sheep-specific antiserum, a pig-specific antiserum, a horse/donkey-specific antiserum, a kangaroo-specific antiserum, a buffalo-specific antiserum, a goat-specific antiserum and a donkey-specific antiserum.

If desired the apparatus may provide for testing for alternative or additional substitutes such as camel or the like.

In an alternative embodiment, a simplified range may include 6 lines of wells coated with 6 specific antigens, antibodies or like materials. The 6 species specific antisera may be a beef/buffalo specific antiserum, a sheep/goat-antiserum, a pig-specific antiserum, a horse/donkey-specific antiserum, a kangaroo-specific antiserum and a buffalo-specific antiserum.

In an alternative embodiment of the present invention the sample to be tested may be a different protein-containing material. A sample may be a milk or milk product. In one form the sample to be tested may be goat's milk which may be adulterated with cow's milk. In this form, only two lines of wells coated with a first and second specific antigen, antibody or like material are required. The first species-specific antigen, antibody or like material may be a cow-specific antiserum. The second species specific antigen antibody or like material may be a goat-specific antiserum. Particularly preferred are a sheep anti-cow antiserum and a sheep anti-goat antiserum.

As stated above the ELISA apparatus accordingly to the present invention includes

a(i)   at least one well in the first line treated to form a control and

a(ii)   at least one well in the second line treated to form a control. Where the ELISA test apparatus includes more than 2 lines of wells each line of wells may include at least one well treated to form a control.

In a preferred embodiment the ELISA apparatus according to the present invention may include (a) one well in each line treated to form a positive control (b) one well in each line treated to form a negative control.

For convenience, the positive and negative controls . may comprise the last two wells in each line.

The positive control well (a) may include a first coating of a specific antigen, antibody or like material and a second coating of a specific anzyme-linked anti-body reagent.

The enzyme linked anti-body reagent may be diluted to provide an optical density approximately 40-50% of an undiluted standard. As discussed below, this solution provides a reaction which generates a colour which is approximately equal to a 1% species content.

The negative control (b) well may comprise a first coating of a specific antibody, antigen or like material. It will be understood that the negative control should provide a negative result to the colour reagent if the system is working properly

In a further aspect of the present invention there is provided a method of assaying a sample which method includes

a)   providing a sample

b)   providing a vessel of the type described above

c)   adding the sample to the at least one coated non-control well of the first line

d)   adding the sample to the at least one coated non-control well of the second line and

e)   adding an enzyme-linked species specific antibody conjugate to the samples in the coated wells of the first and second lines.

In a preferred form, the assay method according to the present invention may further comprise

f)   treating the product of step (e) with a substrate for the enzyme-linked antibody conjugate.

As stated above the sample to be assayed may be any protein-containing material suitable for use with an ELISA method.  The sample may be a meat or a milk, or a product thereof.  The sample may comprise serum components of the sample or muscle constitutents.

In the case of milk or milk products these may be directly tested. It will be understood that the sensitivity of the ELISA method enables the detection of proteins, such as serum albumin and immunoglobbulim directly in the milk although they are present at relatively low levels.

The sample may be prepared as follows. A sample may be extracted with distilled water. For example approximately 50 grams of meat may be extracted with approximately 50 mls of distilled water by soaking for a sufficient period. Soaking may continue for 5 minutes or greater. Alternatively, the extraction may be conducted by stomaching in a plastic bag for an effective period. Approximately 10 seconds is normally adequate.

Sample extract concentrations are not critical. A ratio of approximately 1 ml per gram of sample may be used.

A number of samples may be combined if desired without substantially affecting the practical outcome of the results. Up to five samples may be combined if desired. It will be understood that an advantage of the method according to the present invention is that the ELISA method is such that the sensitivity of the test allows for detection of a contaminent in amount of approximately 0.1% or more. If the presence of an extranious species is detected then individual samples may be tested to determine which of the bulk samples contains the substituted species.

As stated above step (c) & (d) of the assay method comprises adding the sample to the coated non-control wells of each line.

In a preferred form the sample addition steps (c) and (d)

0177352

may comprise

(i)     adding one drop of sample extract to each coated non-control well in each line.

The drops may be delivered utilizing a Pasteur pippette. The size of the droplets is not critical. Drops having a volume in the range of approximately 30 to 60 ul will give approximately equivalent results. Accordingly, the Pasteur pippette may be selected to provide approximately 30 ul drops.

The sample addition steps (c) and (d) may further comprise

(ii)     Incubating the coated vessel for a period sufficient to allow the specific antibody in the sample to become attached to the species-specific antigen, antibody or like material.

The incubation step may be conducted at approximately room temperature. The incubation step may continue for approximately 30 minutes. A lid may be placed on the vessel during incubation as described below.

The sample addition steps (c) and (d) may further comprise

(iii)     emptying the vessel and washing same in water.

The vessel may be washed three times or more using clean water.

Step (e) of the assay method according to the present invention provides for adding an enzyme-linked species-specific antibody conjugate to the samples in the coated wells in the first and second lines of the vessel. Other species specific conjugates are added to the coated non-control wells of any subsequent lines of the vessel.

The conjugate will vary as to

1.      The type of serum used for immunization and

2.      The animal species which is immunized eg. sheep, rabbit, cow etc.

The enzyme may be selected from any of those suitable for use in the ELISA method. For example the enzyme may be an alkaline phosphatase, a glucose oxidaze or a horseradish peroxidaze. The enzyme preferred is horseradish peroxidaze.

The enzyme-antibody conjugate may be preferred in any suitable manner. The preferred method of preparation of the enzyme-antibody conjugate is that of Wilson and Nakane (immunofluorescence and related staining techniques. Editors W. Knapp, K. Holubar and G. Wick, Amsterdam, Elsevier, pp. 215-225, 1978).

The conjugates are selected to correspond to each of the extraneous species sought to be identified. Thus for the identification of extraneous  species in goats milk, the two conjugates may be sheep antigoat IgG-horseradish peroxidase and sheep anti-cow IgG-horseradish peroxidase.

The enzyme-antibody conjugates may then be titrated to provide preferred dilutions. The preferred dilution is one which results in an optical density with the enzyme substrate discussed below of at least 1.0. These dilutions will vary between conjugates and between batches of conjugates so are the subject of simple experimentation. For example, for a beef/ buffalo-specific reagent a dilution of 1:400 may be used for a kangaroo-specific reagent a dilution of approximately 1:600 may be used.

The conjugate addition step (e) may comprise

0177352

(i)      Adding one drop of enzyme-linked species-specific antibody conjugate to the or each coated non-control well of each line corresponding to the species to be identified.

The enzyme-antibody conjugates may be prepared in an aqueous solution. The aqueous solution may be a buffer solution. The buffer solution may be a phosphate buffered saline. The phosphate buffered saline solution may further contain a surfactant. The surfactant may be a polysorbate. The polysorbate may be that sold under the trade designation "Tween twenty".

The buffer solution may further comprise enzyme-antibody conjugate stabilizers. The stabilizers may be selected from ovalbumin, methiolate and phenyl methyl sulphonyl fluoride (PMSF) or mixture thereof. The buffer solution may further comprise an inert dye. The inert dye acts as a visual aid during the addition of the connugates. Bromophenyl blue may be used as the inert dye.

The conjugate addition step (e) may further comprise

(ii)     Incubating the coated vessel for a period sufficient to allow reaction between the conjugate and captured antibody.

The incubation may continue at approximately room temperature for a period of approximately 30 minutes.

As stated above the ELISA assay method may further comprise the further step (f) which comprises

(i)      treating the product of step (e) with a substrate. For the enzyme-linked antibody conjugate step (f) allows for a qualitative and/or quantitative measurement of the amount of antigen  produced.

The substrate may be added to each well of each line of the vessel.

Step (e) may further comprise the further step of (ii)    Allowing the vessel to stand for a period sufficient to allow reaction between the product of step (e) and the enzyme substrate.

The vessel may be allowed to stand for approximately 10-20 minutes.  It will be understood that the development of a blue colour in the embodiment described above characterizes a positive reaction.  By comparison with the colour intensity in the 1% positive control well a qualitative measurement may be made as to whether the extraneous species is or is not present.  A quantitative measurement may be achieved, if desired, by any brown method eg.  measurement of optical density or titration.

The selection of enzyme substrate will be related to the selection of enzyme.  For example, for horseradish peroxidase, an o-tolidine solution may be used.  The solution may be an aqueous solution.  The aqueous solution may be buffered to a pH of approximately 4.5.  The buffer solution may comprise hydrogen peroxide ($H_2O_2$) in sodium citrate. Approximately 2.5 mM $H_2O_2$ in approximately 50 mM sodium citrate buffer may be used.  The o-tolidine may be present in a concentration of approximately 2.5 mM.

One or two drops of the substrate solution may be added to each well on the vessel.  Prior to the addition of the substrate the plate may be washed using a suitable wash solution.  As stated above drop sizes are not critical.  Crop sizes within the range of approximately 25 ul to 60 ul give . acceptable results in the system described.

In a still further aspect of the present invention there is provided a method of preparing a vessel for use in a method for assaying a sample which includes

(a)      providing a vessel including

     (i) a first line of wells and

     (ii)a second line of wells

(b)      coating at least one of the wells of the first line
with a first species-specific antigen, antibody or like
material.

(c)      coating at least one of the wells of the second
line with a second species-specific antigen, antibody or like
material

(d)      treating at least one well in the first line to form
a control and

(e)      treating at least one well in the second line to form
a control.

     The vessel provided in step (a) of the method of
preparation according to the present invention may be a
microtitre plate.  The microtitre plate may be of the type
described above.

     The coating steps (b) and (c) may comprise

(i)      Treating the at least one well of the first line with
a first species-specific antigen, antibody or like material
in a buffer solution

(ii)      Incubating the coated vessel for a period sufficient
to allow the specific antigen to become attached to the well
wall and

(iii)      Washing the coated vessel in a saline solution.

     The species-specific antigen, antibody or like material
may be prepared in 50 microlitre volumes.  The species specific
antibody may be diluted to approximately 2 ug/ml in a buffer
solution.  The buffer solution may be a phosphate buffer saline

(PBS) solution the buffer solution may have a pH of approximately 7.2.

The incubation step (ii) may be conducted at room temperature. The incubation may continue for approximately 12-24 hours. preferably 16 hours.

The washing step (iii) may be conducted utilizing a phosphate buffered saline solution. The saline solution may contain a surfactant. The surfactant may be a polysorbate surfactant. The polysorbate may be that sold under the trade designation "Tween 20".

It will be understood that for each of the species-specific antigens the coating step is repeated. The incubation and washing steps may be undertaken after each line of wells has been coated.

The vessels may be covered utilizing any suitable sealer. A self adhesive plastic sealer may be used. The vessles may be stored at low temperatures. eg. approximately 4°C. It will be understood that the vessels remain stable and active fur approximately 6 months under these conditions.

As specified above the preparation method further control comprises

(d)　　treating at least one well in the first line to form a control. Preferably the control is a negative control. For a negative control the coating step (b) may be repeated on the control well.

In a preferred form the control treatment steps (d) and (e) may form a positive control. Control treatment steps (d) and (e) may comprise

(i)　　repeating step (b) on a second well of the first line, and

(ii)     coating the second well of the first line with a species-specific antibody-enzyme conjugate.

The species specific antibody-enzyme conjugate may be selected as described above. The conjugate coating step may be conducted in a manner similar to that described above.

It will be understood that where there are more than two well lines in the vessel, the coating and control treatment steps may be repeated.

In a still further aspect of the present invention there is provided a test kit for assaying a sample which test kit includes

a)  an enzyme-linked immunosorbent assay apparatus of the type described above

b)  a plurality of species-specific antibody-enzyme conjugates in suitable containers and

c)  an enzyme substrate in a suitable container.

In a preferred form the ELISA apparatus includes a vessel including

a)      a first line of wells

b)      a second line of wells.

Each line of wells may further include III an identity marking. The identify marking may comprise a colour code. It would be understood that a different colour may be selected for each  species to be identified.

Desirably, each container (b) including a species-specific antibody enzyme conjugate also includes a corresponding identity marking. Again a colour coding may be used.

The present invention will now be further described with reference to the accompanying drawings and examples. It should be understood however, that the description following

-16-

**0177352**

is illustrative only and should not be taken in any way as a restriction on the generality of the invention described above.

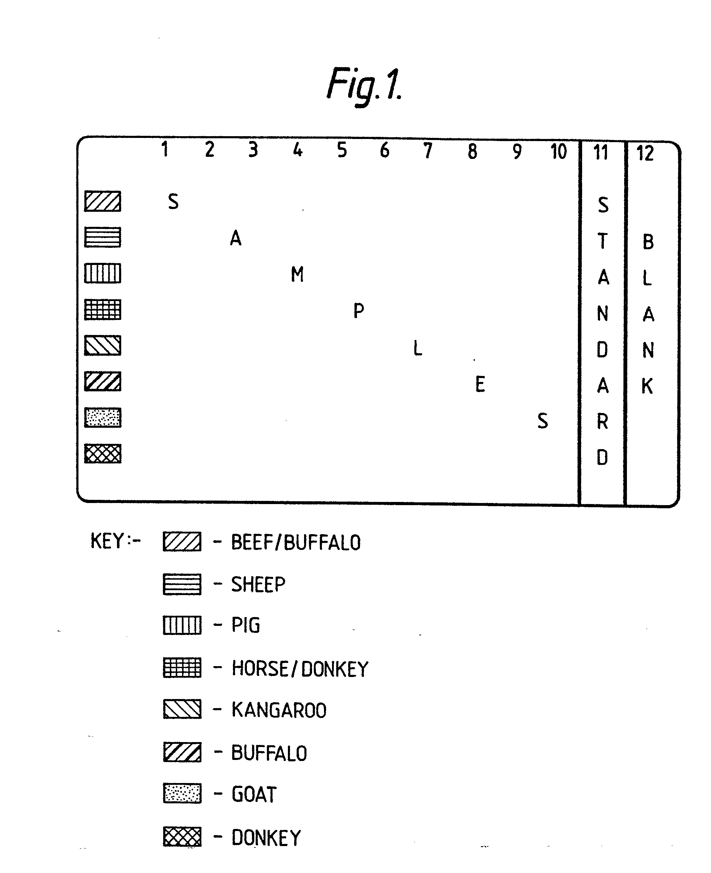

In the accompanying drawings, Figure 1 illustrates an embodiment of a microtitre plate according to the present invention including 8 lines of wells coated with 8 specific antigens. The microtitre plate of Figure 1 allows for testing of 10 samples on the single plate and includes inbuilt positive (standard) and negative (blank) controls on each line.

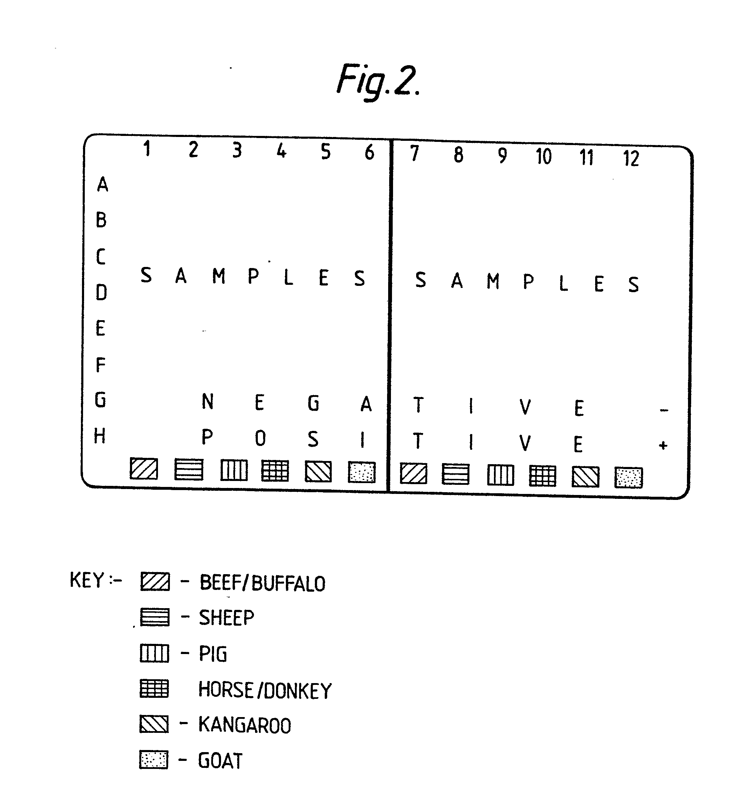

Figure 2 illustrates an embodiment of a microtitre plate according to the present invention. The microtitre plate includes 6 lines of wells coated with 6 specific antigens in a duplicate arrangement. It will be understood that in this embodiment a cover may be placed over the microtitre plate. The cover may be slitted or otherwise perforated between lines 6 and 7 so that only one side of the microtitre plate may be exposed at a time.

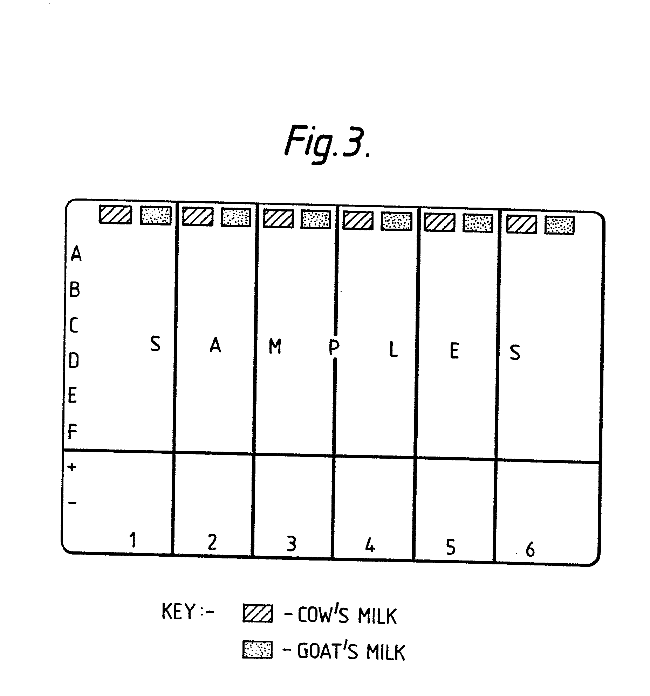

Figure 3 illustrates an embodiment of the microtitre plate according to the present invention including 2 lines of wells coated with two specific antigens in a multiple use format. Once again a cover may be used in this embodiment and removed from each section as required.

Examples

Antiserum Production

Depending on the species to be tested for, antisera are raised in either sheep, goats, horses, donkeys, cattle or any other appropriate animal. Typical antiserum and their extraction are illustrated in Table 1. For example, the specific anti-sheep reagent was made by immunising a goat with 20 mg of whole sheep serum in a multiple emulsion of Freunds adjuvant. As prepared the antiserum from this goat had a

0177352

relatively low titre.  The reason for this is that sheep and goats are closely related animals and thus sheep serum is not seen as being particularly foreign to the goat immune system.

For this reason, in all cases specific antibodies are purified from whole antiserum using accepted affinity chromatography procedure.  Although this would appear to be straightforward a number of problems were encountered.  For example, the goat anti-sheep produced a very slight cross-reaction with beef.  This is to be expected as both beef and sheep are ruminants and although not as closely related as sheep and goats, there is a degree of overlap.  This cross-reaction was removed by adsorption on a 'beef' serum affinity column.

Secondly, it was observed that in some cases anti idiotypic antibody was produced and this was responsible for false-positive reactions.  This could be removed by passing the appropriate affinity extracted antibody through a serum affinity column of the same species from which that antibody was prepared, e.g. goat anti-sheep would have to be passed through a goat serum affinity column.

These are just two examples of the problems encountered in producing specific and non-cross-reacting antisera for use in these systems.  Similar problems were encountered with each species.  All antisera were produced by immunising animals using whole serum proteins from the species in question.  Purified serum albumin or serum immunoglobulin, for example, also produce workable reagents but they do not appear to be as good as those obtained using whole serum proteins as the immunogen.  The latter provides a greater range of antigenic epitopes which are particular useful in differentiating closely related species, e.g. sheep/goat.

TABLE 1

PREPARATION OF REAGENTS FOR MEAT SPECIATION ELISA

ANTI-COW:     -  Sheep anti-cow serum.

REAGENT:         Specific antibody was affinity extracted on
                 bovine serum affinity columns.

ANTI-SHEEP:   -  Coat anti-sheep serum.

REAGENT:         Crude antisera was passed through a bovine
                 serum affinity column and specific antibody
                 was then affinity extracted on a sheep serum
                 affinity column.

ANTI-PIG:     -  Sheep anti-pig serum.

REAGENT:         Specific antibody was affinity extracted on a
                 pig serum affinity column.

ANTI-HORSE:   -  Cow anti-horse serum.

REAGENT:         Specific antibody was affinity extracted on a
                 horse serum affinity column.

ANTI-KANGAROO-  Cow anti-kangaroo serum.

REAGENT:         Specific antibody was affinity extracted on a
                 kangaroo serum affinity column.

ANTI-BUFFALO:-  Cow anti-buffalo serum

REAGENT:         Crude antisera was passed through a bovine serum
                 affinity column and specific antibody affinity
                 extracted on a buffalo serum affinity column.

ANTI-GOAT:       Sheep anti-goat serum.

REAGENT:         Specific antibody was affinity extracted on a
                 goat serum affinity column.

ANTI-DONKEY:  -  Horse anti-donkey.

REAGENT:         Specific antibody was affinity extracted on a
                 donkey serum affinity column.

TABLE 1 continued

PLATE SEALERS:      LINBRO[R]/TITERTEK[R]

adhesive backed Acetate plate sealer

(13.2 cm x 8.2 cm)   CAT.NO. 76-40]-05

Example 1

Speciating of Meat

STEP 1   Sample preparation

Samples are extracted by soaking in distilled water for a minumum of 5 minutes or 'stomaching' in a plastic bag for 10 seconds. Sample extract concentrations are not critical although a ratio of approximately 1 ml per gram meat is recommended. Sample extracts can be bulked if desired, (no more than 5 samples per test).

STEP 2   Sample addition.

Sample extracts are added (one drop per well) using a disposable plastic or glass pasteur pipette direct from the sample container, DOWN each column. Columns 1-10 are used for test samples, columns 11 and 12 provide 1% species detection standards and blank (background) controls respectively (sample extracts should NOT be added to columns 11 and 12). A lid is placed on the plate(s) and it is incubated at room temperature for 30 minutes.

STEP 3   Plate washing.

Shake plate out in sink. Wash at least three times using clear water, e.g. under a gently running tap. Shake out between washes and tap plate firmly upside down on a paper towel after last wash.

STEP 4   Conjugate addition.

Species-specific antibody-enzyme conjugates are provided in colour-coded drop bottles. Add one drop per well horizontally ACROSS the row corresponding with the species, ensuring that the colour codes are observed, e.g. red coded conjugate is added across the red coded row of the plate. All conjugates contain an inert blue dye to facilitate correct addition of drops. Plates are then covered with a lid and incubated at room temperature for 30 minutes.

STEP 5    Plate washing as in step 3.

STEP 6    Substrate addition.

Substrate is provided in labelled white-topped bottles (colour reagent). Two drops of this colourless substrate solution are then added to each of the 96 wells on the microtitre plate.

STEP 7    Interpretation.

Plates are examined after 10 to 20 minutes for the development of a blue colour which characterizes a positive reaction. Al standards must be positive and the blank wells colourless for the test to be valid. A positive reaction is defined as a well which has developed a blue colour equal to or greater than the intensity in the 1% positive control well.

NOTE:-    Drop sizes within the range 25 ul to 60 ul give acceptable results in the system described.

Example 2

Speciation of Milk

STEP 1    Sample preparation

No sample preparation is necessary. Whole OR diluted milk samples may be tested.

STEP 2    Sample addition

The sealing tape is removed from the appropriate wells prior to use. Samples are added (ONE drop per well) to the required number of cow (red) and goat (brown) wells using a disposable plastic pasteur pipette. Additions should NOT be made to the rows marked '+' and '-' at this stage. A lid is placed on the plate and it is kept at room temperature for 5-10 minutes.

STEP 3    Plate washing

Shake the liquid out of the wells in a sink. Wash at least three times using clean water, e.g. under a gently running tap. Shake out between washes and tap plate firmly upside down on a paper towel after the last wash.

STEP 4    Specific reagent addition

Cow and goat-specific reagents are provided in colour-coded drop bottles. Add ONE drop per well from the appropriate bottle to the required number of wells, ensuring that the colour codes are observed, i.e. 'red' reagent to the 'red' wells and 'brown' reagent to the 'brown' wells. These reagents are also added to the '+' and '-' wells at the bottom of the plate. A lid is then placed on the plate and it is incubated at room temperature for 5-10 minutes.

STEP 5    Plate washing as in STEP 3

STEP 6    Colour development

The colour reagent is provided in labelled white-topped bottles. TWO drops of this colourless solution are then added to the appropriate wells.

STEP 7    Interpretation

The wells are examined after 5-10 minutes for the development of a positive reaction which is defined as a distinct blue colour. Detection of colour is facilitated by placing the plate on a white background.

Controls are provided on the plate in the form of positive and negative wells. The positive wells have been pretreated to give a reaction which approximates that shown by a 1% sample of that milk. It is suggested that these wells are included in the testing regime every time a sample or series of samples are assayed. The system is working correctly when a distinct blue colour is observed in the '+' wells and the '-' wells remain clear.

The following were obtained with the kit reagents. All determinations were performed in quadruplicate and the colour produced was quantitated using an ELISA plate reader. For clarity the readings are expressed as the optical density x 100 ± S.D.

COW SYSTEM:-

|  |  |
|---|---|
| 100% cow's milk | 135 ± 4 |
| 10% cow's milk | 143 ± 1 |
| 1% cow's milk | 133 ± 2 |
| 0.1% cow's milk | 116 ± 5 |
| 0.01% cow's milk | 45 ± 4 |
| 100% goat's milk | 5 ± 2 |

GOAT SYSTEM:-

|  |  |
|---|---|
| 100% goat's milk | 164 ± 6 |
| 10% goat's milk | 156 ± 6 |
| 1% goat's milk | 110 ± 6 |
| 0.1% goat's milk | 30 ± 2 |
| 0.01% goat's milk | 23 ± 2 |
| 100% cow's milk | 12 ± 4 |

The above were constituted by the dilution of the appropriate volume of milk from the species in question in milk from the complementary species, e.g. one volume of cow's milk in 99 volumes of goat's milk to make 1% cow's milk.

Finally it is to be understood that various other modification and/or alterations may be added to the specification without departing from the spirit of the present invention as outlined herein.

CLAIMS:

**0177352**

1.      An apparatus for enzyme-linked immunosorbent assay including

(1)   a vessel including

(a)   a first line of wells; and

(b)   a second line of wells;

the first line of wells (a) including

(i)   at least one well adapted to form a control, and

(ii) at least one well coated with a first specific antigen, antibody or like material;

the second line of wells (b) including

(i)   at least one well adapted to form a control, and

(ii) at least one well coated with a second specific antigen, antibody or like material; each specific antigen, antibody or like material being selected to recognize and bind to a sample.

2.      An apparatus according to claim 1 wherein the vessel further includes

(c)   at least one further line of wells including

(i)   at least one well adapted to form a control, and

(ii) at least one well coated with a further specific antigen, antibody or like material.

3.      An apparatus according to claim 2 wherein each specific antigen, antibody or like material is selected from antisera including beef/buffalo- specific antiserum, a sheep-specific antiserum, a pig-specific antiserum, a horse/donkey-specific antiserum, a kangaroo-specific antiserum, a buffalo-specific antiserum, a goat-specific antiserum and a donkey-specific antiserum or mixtures thereof.

4.     An apparatus according to claim 2 wherein the sample to be tested is a milk or milk product and the antisera are selected from sheep anti-cow antiserum and sheet-anti-goat antiserum.

5.     An apparatus according to claim 2 wherein one well in each line is treated to form a positive control and (b) one well in each line is treated to form a negative control;

the positive control well (a) including a first coating of a specific antigen, antibody or like material.

6.     An apparatus for an enzyme-linked immunosorbent assay including

(1)    a first vessel according to claim 2,

(2)    at least one further vessel adjacent thereto,

(3)    a first dividing member between adjacent vessels, and

(4)    a second dividing member between the at least one coated well and the control well.

7.     A method of assaying a sample which method includes

(a)    providing a sample,

(b)    providing an apparatus as defined in claim 1,

(c)    adding a portion of the sample to be tested to at least one coated  non-control well of the first line,

(d)    adding a portion of the sample to the at least non-coated non-control well of the second and any subsequent lines,

(e)    adding an enzyme-linked species-specific antibody conjugate to the sample wells and control wells of the first and second lines, and

(f)    treating the product of step (e) with a substrate for the enzyme-linked antibody conjugate.

8.    A method according to claim 7 wherein the sample addition steps (c) and (c) include

(i)  adding one drop of sample extract to each coated non-control well in each line,

(ii)  incubating the coated vessel for a period sufficient to allow the specific antibody in the sample to become attached to the species-specific antigen, antibody or like material; and wherein the conjugate additions to (e) includes

(i)  adding one drop of enzyme-linked species-specific antibody conjugate to the or each coated non-control well of each line corresponding to the species to be identified, and

(ii)  incubating the coated vessel for a period sufficient to allow reaction between the conjugate and captured antibody.

9.    The method of preparing a vessel for use in a method for assaying a sample which includes

(a)   providing a vessel including

(i)  a first line of wells, and

(ii) a second line of wells,

(b)   coating at least one of the wells of the first line with a first species specific antigen, antibody or like material,

(c)   coating at least one of the wells of the second line with a second species-specific antigen, antibody or like material,

(d)   treating at least one well in the first line to form a control, and

(e)   treating at least one well in the second line to

form a control.

0177352

10. A method according to claim 9 wherein the coating steps (b) and (c) include

(i) treating the at least one well of the first line with a first species specific antigen, antibody or like material in a buffer solution.

11. A method according to claim 10 wherein step (d) includes treating at least one well in the first line to form a positive or negative control.

12. A method according to claim 11 wherein the control is a positive control and treatment step (d) includes

(i) repeating step (b) on a second well of the first line and

(ii) coating the second well of the first line with a coating standard being an assay sample of known composition.

13. A test kit for assaying a sample which test kit includes

(a) an apparatus for enzyme-linked immunosorbent assay according to claim 1,

(b) a plurality of species-specific antibody-enzyme conjugants in suitable containers, and

(c) an enzyme substrate in a suitable container.

0177352

# Fig.1.

|  | 1 | 2 | 3 | 4 | 5 | 6 | 7 | 8 | 9 | 10 | 11 | 12 |
|---|---|---|---|---|---|---|---|---|---|---|---|---|
| ▨ | S | | | | | | | | | | S | |
| ▤ | | A | | | | | | | | | T | B |
| ▥ | | | | M | | | | | | | A | L |
| ▦ | | | | | | P | | | | | N | A |
| ◨ | | | | | | | | L | | | D | N |
| ▨ | | | | | | | | | E | | A | K |
| ▩ | | | | | | | | | | S | R | |
| ▩ | | | | | | | | | | | D | |

KEY:-  ▨ – BEEF/BUFFALO

▤ – SHEEP

▥ – PIG

▦ – HORSE/DONKEY

◨ – KANGAROO

▨ – BUFFALO

▩ – GOAT

▩ – DONKEY

# Fig.2.

| | 1 | 2 | 3 | 4 | 5 | 6 | 7 | 8 | 9 | 10 | 11 | 12 |
|---|---|---|---|---|---|---|---|---|---|---|---|---|
| A | | | | | | | | | | | | |
| B | | | | | | | | | | | | |
| C | | | | | | | | | | | | |
| D | S | A | M | P | L | E | S | S | A | M | P | L E S |
| E | | | | | | | | | | | | |
| F | | | | | | | | | | | | |
| G | | N | E | G | A | T | I | V | E | | | − |
| H | | P | O | S | I | T | I | V | E | | | + |

KEY :-

| ▨ | – BEEF/BUFFALO |
|---|---|
| ▤ | – SHEEP |
| ▥ | – PIG |
| ▦ | HORSE/DONKEY |
| ▧ | – KANGAROO |
| ▨ | – GOAT |

0177352

# Fig.3.

| | | | | | | |
|---|---|---|---|---|---|---|
| | ▨ ▦ | ▨ ▦ | ▨ ▦ | ▨ ▦ | ▨ ▦ | ▨ ▦ |
| A B C D E F | S | A | M P | L | E | S |
| + − | | | | | | |
| | 1 | 2 | 3 | 4 | 5 | .6 |

KEY :− ▨ – COW'S MILK

▦ – GOAT'S MILK

## DOCUMENTS CONSIDERED TO BE RELEVANT

EP 85307079.5

| Category | Citation of document with indication, where appropriate, of relevant passages | Relevant to claim | CLASSIFICATION OF THE APPLICATION (Int Cl.4) |
|---|---|---|---|
| X | CH - A5 - 613 523 (INSTITUT NATIONAL DE LA SANTE ET DE LA RECHERCHE MEDICALE)<br>* Example 5; fig. * | 1,2,6 | G 01 N 33/545<br>G 01 N 33/535<br>G 01 N 33/04 |
| X | EP - A1 - 0 084 102 (BIOTEST-SERUM-INSTITUT GMBH)<br>* Claim 1; fig. 1 * | 1,2,6 | |

**TECHNICAL FIELDS SEARCHED (Int. Cl.4)**

G 01 N 33/00

The present search report has been drawn up for all claims

| Place of search | Date of completion of the search | Examiner |
|---|---|---|
| VIENNA | 04-12-1985 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82